# EUROPEAN PATENT APPLICATION

(11) **EP 3 167 959 A1**
(43) Date of publication of application: **17.05.2017**
(21) Application number: 16797707.3
(22) Date of filing: 07.07.2016
(51) Int. Cl.: B01L 1/00, A61L 2/20, F24F 7/06, A61L 101/10

(54) **SAFETY CABINET AND METHOD FOR DECONTAMINATING SAFETY CABINET**

(30) Priority: 07.07.2015 JP 2015135723
(71) Applicant: Tamura Teco Co. Ltd., Higashi-Osaka-shi, Osaka 577-0012 (JP)
(72) Inventor: WATANABE Naoki, Souka-shi Saitama 340-0002 (JP); SHU Raku, Souka-shi Saitama 340-0002 (JP); TAMURA Kozo, Higashi-Osaka-shi Osaka 577-0012 (JP); NISHIZAWA Hiroyuki, Higashi-Osaka-shi Osaka 577-0012 (JP)
(74) Representative: Lemcke, Brommer & Partner Patentanwälte Partnerschaft mbB
(86) International application number: PCT/JP2016/070101
(87) International publication number: WO 2017/006983

(57) **Abstract**

Provided is a safety cabinet capable of preventing ozone gas from leaking out of a working chamber during decontamination and circulating the ozone gas in the working chamber to decontaminate the working chamber.

A cabinet main body (1); a shutter (4) that allows opening/closing of an opening part (3) communicatively connecting to a working chamber (2) ; an exhaust path (6) through which gas is exhausted from the working chamber (2) : an exhaust valve (B2) that is provided in the exhaust path (6); an air supply and circulating fan (P1) that is provided in the working chamber (2) ; an ozone generator (13) that introduces ozone gas into the working chamber (2) ; a control part (10) that controls the exhaust valve (B2), air supply and circulating fan (P1), and ozone generator (13); and airtightly closing means (30) that allows the shutter (4) to airtightly close the opening part (3) are included, and the gas in the working chamber (2) is made internally circulatable, so that the ozone gas can be prevented from leaking out, and the ozone gas can be internally circulated in the working chamber to decontaminate the working chamber with the ozone gas at a set CT value.

## Description

### Technical Field

The present invention relates to a safety cabinet used in fields of industry such as medical care, regenerative medicine, and pharmaceutical, and to a method for decontaminating the safety cabinet.

### Background Art

A safety cabinet is formed therein with a working chamber in a substantially sealed state except for a work opening part; draws contaminated aerosol produced in the working chamber to prevent the contaminated aerosol from flowing out to an operator side as well as including a function of sterilizing and cleaning the drawn and collected contaminated air with a HEPA filter and then exhausting the sterilized and cleaned air; and is classified into classes I, II, and III depending on the level of a pathogen to be treated.

As an example of such a safety cabinet, one described in Patent Literature 1 is known. This safety cabinet has an opening/closing door at the front, and includes: a cabinet main body formed therein with a working chamber; a high-performance air supply filter provided on one side of the working chamber; an air blower adapted to compressively transfer air to the high-performance air supply filter; a working table that is provided on the other side of the working chamber and has exhaust ports through which air inside the working chamber passes; a communicatively connecting path through which the air blower draws air flowing out of the working chamber through the exhaust ports; and a discharge path that is provided leeward of the air blower and through which air is discharged to the outside of the cabinet main body via a high-performance exhaust filter.

Also, the communicatively connecting path is provided with an ozone generator, whereas the discharge path is provided with an ozone removing member, and it is adapted to, in a state of closing the opening/closing door, activate the ozone generator and operate the air blower at low speed equal to or less than the rated rotation speed, and in a state of stopping the ozone generator, operate the air blower at the rated rotation speed.

In such a safety cabinet, when after the end of work, activating the ozone generator to generate ozone gas in the state of closing the opening/closing door, the generated ozone gas is circulated through the communicatively connecting path by the air blower, and this makes it possible to sterilize (decontaminate) the communicatively connecting path outside the working chamber. In particular, since during the action of the ozone generator, the air blower is operated at low speed equal to or less than the rated rotation speed, wasteful ozone gas generation can be suppressed and at the same time a relatively wide range can be sterilized.

Further, since after stopping the ozone generator to end the sterilization (decontamination) with the ozone gas, the air blower is operated at the rated rotation speed, the ozone gas contained in air inside the cabinet main body is discharged via the high-performance exhaust filter, and at this time as well, since the discharge path is provided with the ozone removing member, the ozone gas can be immediately removed to prevent the ozone gas from being discharged outside together with exhaust.

In the working chamber of such a safety cabinet, the preparation work of an anticancer drug, hormone drug, antibiotic drug, or the like is also performed. In addition, after the end of the preparation work, the anticancer drug, hormone drug, antibiotic drug, or the like may be suspended in the working chamber and/or attached on the inner wall surfaces of the working chamber as a residue, and therefore also in order to prevent the residue from rescattering, it is necessary to decontaminate the residue.

### Citation List

### Patent Literature

[Patent Literature 1]
Japanese Unexamined Patent Publication JP-A7-8811

### Summary of Invention

### Technical Problem

Meanwhile, ozone gas is known to have an effect of decomposing and removing an anticancer drug, or the like, and in the conventional safety cabinet, the communicatively connecting path outside the working chamber can be decontaminated with the ozone gas.

However, the ozone gas generated by the ozone generator is not supplied to the working chamber, and therefore it is difficult to completely decontaminate the residue suspended in the working chamber and/or the residue attached on the inner wall surfaces of the working chamber.

1 Accordingly, when introducing ozone gas into the working chamber of the above-described conventional safety cabinet, the residue in the working chamber can be decontaminated; however, in this case, it is necessary to, during the decontamination, seal the working chamber so as to prevent the ozone gas from leaking outward of the working chamber (safety cabinet) as well as circulating the ozone gas in the working chamber.

However, in the conventional safety cabinet, it is difficult to, during the decontamination with the ozone gas, prevent the ozone gas from leaking out of the working chamber and circulate the ozone gas in the working chamber, and it is also difficult to perform the decontamination with a predetermined concentration of the ozone gas.

The present invention is made in consideration of the situations, and the object thereof is to provide a safety cabinet capable of, during decontamination with a gaseous decontamination agent such as ozone gas, preventing the gaseous decontamination agent from leaking out of a working chamber and circulating the gaseous decontamination agent in the working chamber to decontaminate the working chamber with the gaseous decontamination agent, as well as decontaminating the working chamber with a predetermined concentration of the gaseous decontamination agent.

### Solution to Problem

In order to accomplish the object, the safety cabinet according to the present invention is a safety cabinet including: a cabinet main body that has a working chamber inside; an opening/closing member that is provided at the front of the cabinet main body and allows opening/closing of an opening part communicatively connecting to the working chamber; and an exhaust path through which gas is exhausted from the working chamber, in which the gas in the working chamber is made internally circulatable, and the safety cabinet includes:
an exhaust valve that is provided in the exhaust path;
an air supply and circulating fan that is provided in the working chamber;
decontamination agent introduction means that introduces a gaseous decontamination agent into the working chamber;
a control part that controls the exhaust valve, the air supply and circulating fan, and the decontamination agent introduction means; and
airtightly closing means that allows the opening/closing member to airtightly close the opening part.

In the present invention, when decontaminating the working chamber with the gaseous decontamination agent, after the control part has closed the exhaust valve and the airtightly closing means has made the opening/closing member airtightly close the opening part, the control part controls the decontamination agent introduction means to introduce the gaseous decontamination agent into the working chamber as well as driving the air supply and circulating fan, and as a result, since the exhaust valve is closed and also the opening part is airtightly closed, the gaseous decontamination agent introduced into the working chamber internally circulates in the working chamber without leaking out of the working chamber.

Accordingly, the gaseous decontamination agent can be prevented from leaking out of the working chamber during the decontamination with the gaseous decontamination agent, and the gaseous decontamination agent can be circulated in the working chamber to decontaminate the working chamber with the gaseous decontamination agent.

Also, in the configuration of the present invention, it is preferable that a concentration sensor adapted to detect the concentration of the gaseous decontamination agent in the working chamber is provided connected to the control part, and on the basis of the concentration of the gaseous decontamination agent detected by the concentration sensor, the control part controls the decontamination agent introduction means.

In such a configuration, since the control part controls the decontamination agent introduction means on the basis of the concentration of the gaseous decontamination agent detected by the concentration sensor, the working chamber can be decontaminated with a predetermined concentration of the gaseous decontamination agent.

Further, in the configuration of the present invention, it is preferable that the control part controls the decontamination agent introduction means on the basis of a CT value that is the product of the concentration of the gaseous decontamination agent detected by the concentration sensor and a decontamination time.

In such a configuration, since the control part controls the decontamination agent introduction means on the basis of the CT value that is the product of the concentration of the gaseous decontamination agent detected by the concentration sensor and the decontamination time, the control part can stop the decontamination agent introduction means when the CT value is equal to or more than a set CT value. Accordingly, the working chamber can be decontaminated with the gaseous decontamination agent in an appropriate time.

Still further, in the configuration of the present invention, it is preferable that the airtightly closing means has an inflatable seal.

In such a configuration, the opening/closing member can airtightly close the opening part communicatively connecting to the working chamber by inflating the inflatable seal, whereas by deflating the inflatable seal, the opening/closing member can be easily moved from the opening part to open the opening part.

Yet further, in the configuration of the present invention, it is preferable that the decontamination agent introduction means is configured to be able to introduce air into the working chamber, and a pressure sensor adapted to detect the internal pressure of the working chamber is provided connected to the control part.

In such a configuration, before decontaminating the working chamber with the gaseous decontamination agent, the control part closes the exhaust valve as well as making the decontamination agent introduction means introduce air into the working chamber to raise the internal pressure of the working chamber, and the raised internal pressure is detected by the pressure sensor. In addition, when the pressure sensor detects that the internal pressure of the working chamber is kept at a predetermined internal pressure for a predetermined time, the control part can start the decontamination agent introduction means to introduce the gaseous decontamination agent into the working chamber. Accordingly, the initial leakage of the gaseous decontamination agent can be prevented.

Also, a method for decontaminating a safety cabinet according to the present invention is a method for decontaminating the safety cabinet, and
the method uses the airtightly closing means to airtightly seal the opening part of the working chamber of the safety cabinet by the opening/closing member as well as closing the exhaust valve, and after the airtightness level of the working chamber has become a predetermined value or more, introduces the gaseous decontamination agent into the working chamber by the decontamination agent introduction means; and
in a state where the concentration of the gaseous decontamination agent rises to a predetermined value, decontaminates the working chamber with the gaseous decontamination agent.

In the present invention, during the decontamination with the gaseous decontamination agent, the gaseous decontamination agent can be prevented from leaking out of the working chamber, and the working chamber can be decontaminated with the predetermined concentration of the gaseous decontamination agent.

Further, in the configuration of the present invention, it is preferable to, when the CT value of the gaseous decontamination agent in the working chamber reaches a predetermined value, stop the introduction of the gaseous decontamination agent.

In such a configuration, since when the CT value of the gaseous decontamination agent in the working chamber reaches the predetermined value, the introduction of the gaseous decontamination agent is stopped, the working chamber can be decontaminated with the gaseous decontamination agent in an appropriate time.

Still further, in the configuration of the present embodiment, it is preferable to, when the concentration of the gaseous decontamination agent in the working chamber is equal to or more than the predetermined value, inflate the inflatable seal to thereby keep unopenable the opening/closing member allowing opening/closing of the opening part communicatively connecting to the working chamber.

In such a configuration, since when the concentration of the gaseous decontamination agent in the working chamber is equal to or more than the predetermined value, the inflatable seal keeps the opening/closing member unopenable, it is impossible for an operator to carelessly open the opening/closing member, thus being superior in safety.

### Advantageous Effects of Invention

According to the present invention, while during the decontamination with the gaseous decontamination agent, preventing the gaseous decontamination agent from leaking out of the working chamber, the gaseous decontamination agent can be circulated in the working chamber to decontaminate the working chamber with the gaseous decontamination agent, and also the working chamber can be decontaminated with the predetermined concentration of the gaseous decontamination agent.

### Brief Description of Drawings

[Fig. 1]

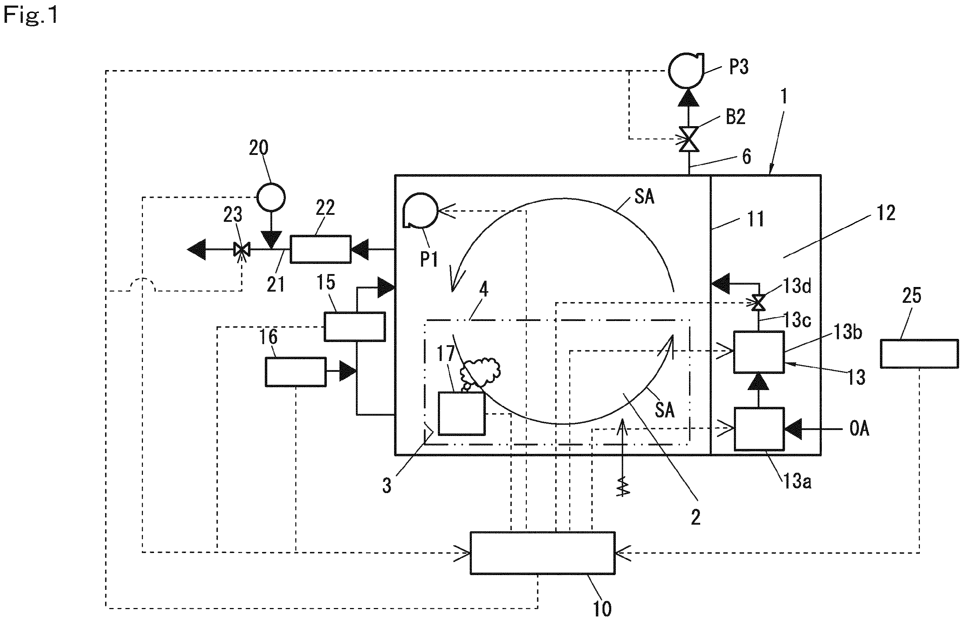

   Fig. 1 is a block diagram illustrating an example of a circulation type safety cabinet according to the present invention, in which the schematic configuration of the safety cabinet is illustrated.
[Fig. 2A]

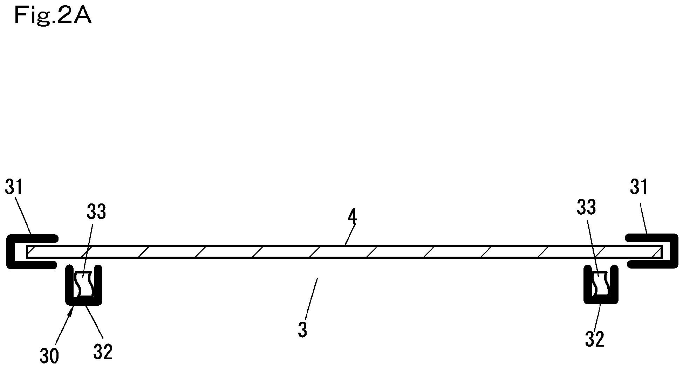

   Fig. 2A is a cross-sectional plan view illustrating the schematic configuration of airtightly closing means for an opening part of a working chamber, in which a state where inflatable seals are deflated is illustrated.
[Fig. 2B]

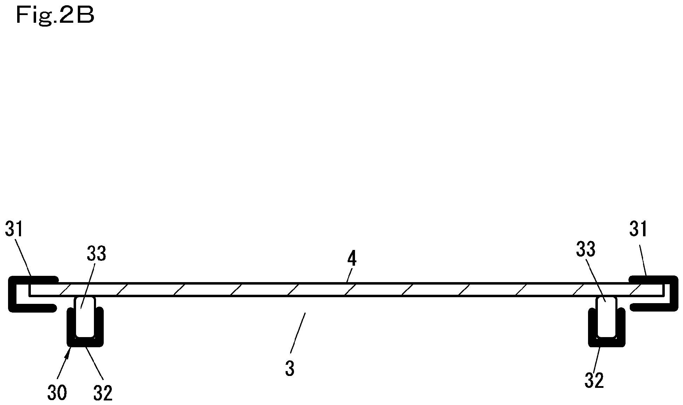

   Fig. 2B is a cross-sectional plan view illustrating the schematic configuration of the airtightly closing means for the opening part of the working chamber, in which a state where the inflatable seals are inflated is illustrated.
[Fig. 2C]

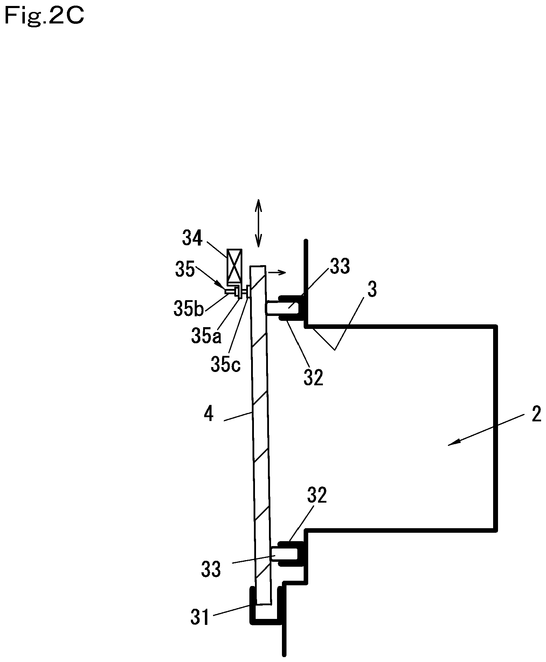

   Fig. 2C is a cross-sectional side view illustrating the schematic configuration of the airtightly closing means for the opening part of the working chamber, in which the state where the inflatable seals are inflated is illustrated.
[Fig. 3]

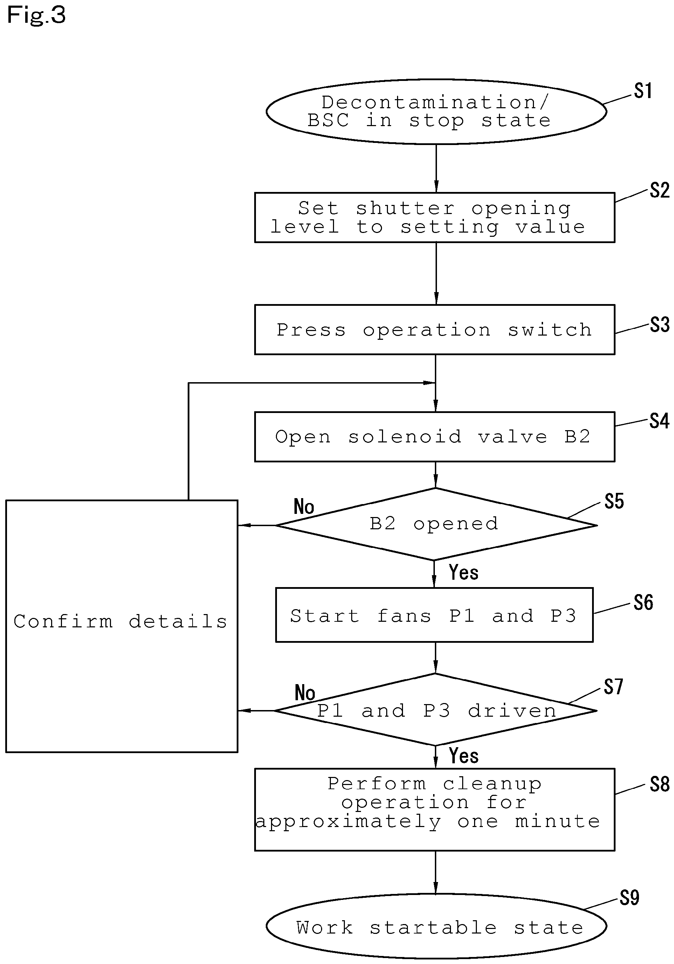

   Fig. 3 is a flowchart for explaining an operation flow of the safety cabinet according to the present invention.
[Fig. 4]

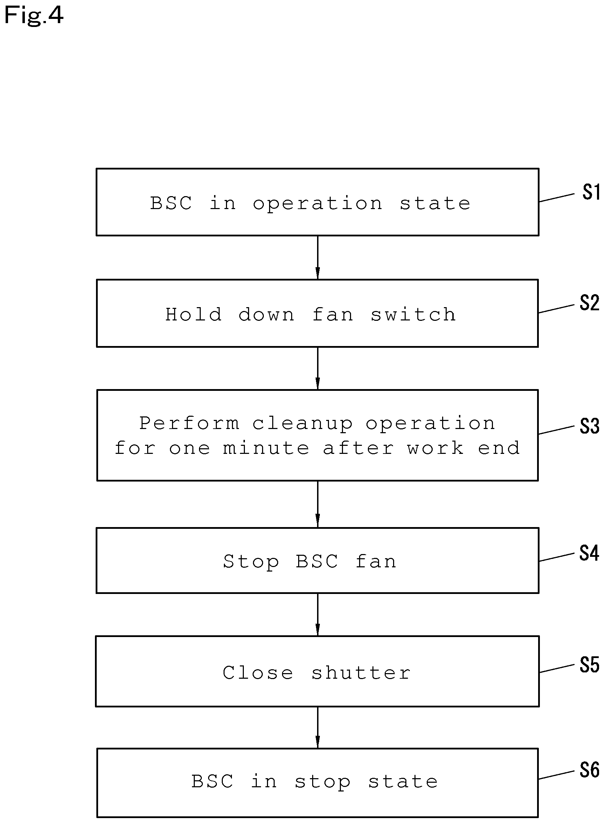

   Fig. 4 is a flowchart for explaining a stop flow of the safety cabinet according to the present invention.
[Fig. 5]

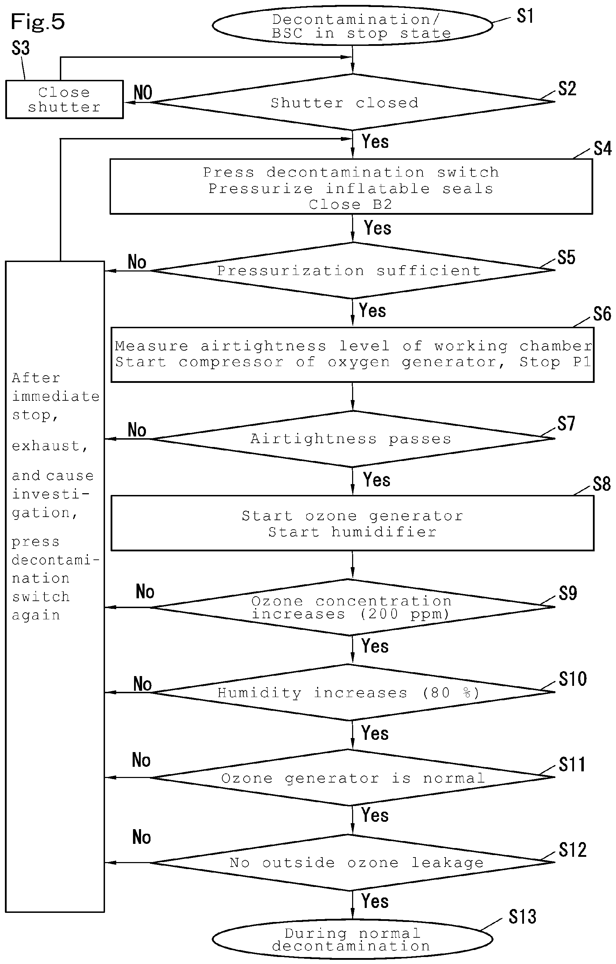

   Fig. 5 is a flowchart for explaining a decontamination operation flow of the safety cabinet according to the present invention.
[Fig. 6]

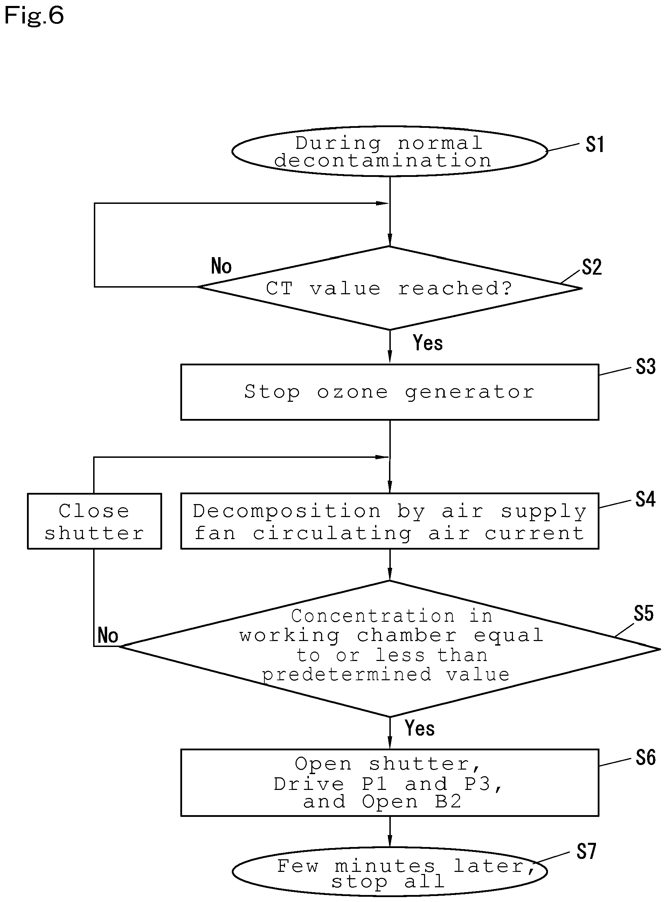

   Fig. 6 is a flowchart for explaining an automatic decontamination stop flow of the safety cabinet according to the present invention.
[Fig. 7]

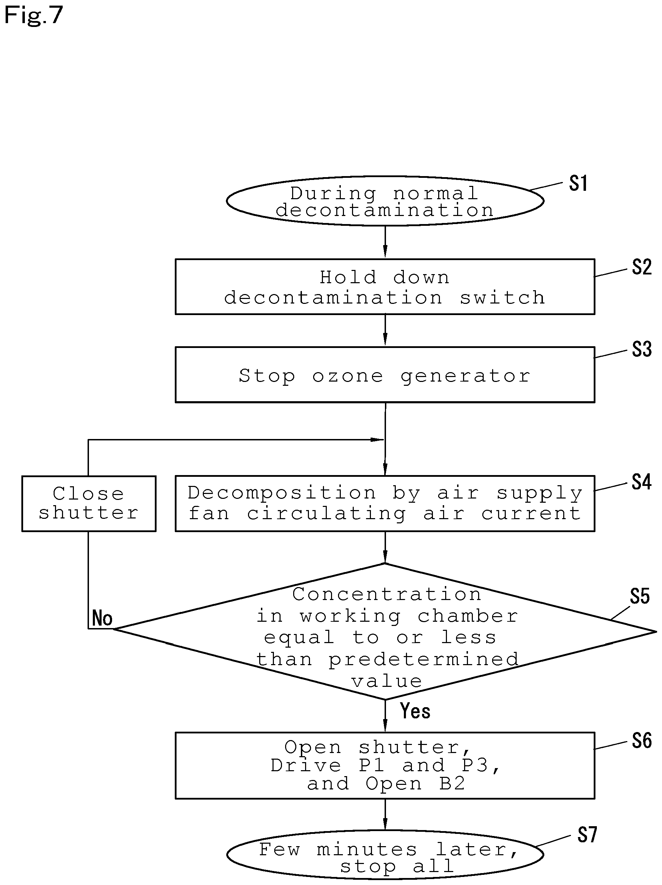

   Fig. 7 is a flowchart for explaining a forcible decontamination stop flow of the safety cabinet according to the present invention.

### Description of Embodiments

In the following, an embodiment of the present invention will be described with reference to the drawings.

Fig. 1 is a block diagram illustrating the schematic configuration of a safety cabinet according to the present embodiment.

As illustrated in Fig. 1, the safety cabinet includes: a cabinet main body 1 having a working chamber 2 inside; a shutter (an opening/closing member) 4 that is provided at the front of the cabinet main body 1 and allows opening/closing of an opening part 3 communicatively connecting to the working chamber 2; and an exhaust path 6 through which gas is exhausted from the working chamber 2.

Note that although illustration is omitted, the cabinet main body 1 is provided with a filter such as a HEPA filter, and the gas exhausted outward of the working chamber through the exhaust path 6 is adapted to be cleaned by the filter.

In addition, the exhaust path 6 is provided with an exhaust valve B2. The exhaust valve B2 includes a solenoid valve, is electrically connected a control part 10, and is adapted to be controlled by the control part 10.

Also, the cabinet main body 1 is provided with an air supply and circulating fan P1. The air supply and circulating fan P1 has a function of drawing air through the opening part 3 to supply the air to the working chamber 2 as well as exhausting the gas in the working chamber 2 outside through the exhaust path 6.

Further, the air supply and circulating fan P1 also has a function of producing a circulating air current SA that causes the gas in the working chamber 2 to internally circulate.

Still further, inside the cabinet main body 1, a partition wall 11 is provided, and the partition wall 11 divides the inside of the cabinet main body 1 into the working chamber 2 and an installation chamber 12. In the present embodiment, ozone gas is used as a gaseous decontamination agent, and therefore an ozone generator (decontamination agent introduction means) 13 is provided in the installation chamber 12.

The ozone generator 13 includes an oxygen generator 13a and an ozonizer 13b, and is adapted such that the oxygen generator 13a takes in the outside air to generate oxygen as well as supplying the oxygen to the ozonizer 13b, and the ozonizer 13b generates the ozone gas (the gaseous decontamination agent).

The ozone gas generated by the ozonizer 13b is adapted to be introduced into the working chamber 2 through an ozone gas supply path 13c. The ozone gas supply path 13c is provided with a supply valve 13d, and it is adapted to supply/stop supplying the ozone gas to the working chamber 2 by opening/closing the supply valve 13d. In addition, the supply valve 13d includes a solenoid valve.

Also, the oxygen generator 13a, ozonizer 13b, and supply valve 13d are electrically connected to the control part 10, and adapted to be controlled by the control part 10.

Further, the ozone generator 13 having such a configuration is configured to be able to introduce air into the working chamber 2 by driving a compressor of the oxygen generator 13a.

In addition, the cabinet main body 1 is provided with: a concentration sensor 15 adapted to detect the ozone concentration of the ozone gas in the working chamber 2; and a thermo-hygro sensor 16 adapted to detect the temperature and humidity of the working chamber 2, and the concentration sensor 15 and the thermos-hygro sensor 16 are electrically connected to the control part 10. Further, the working chamber 2 is provided with a humidifier 17, and the humidifier 17 is electrically connected to the control part 10.

Also, the cabinet main body 1 is provided with a pressure sensor 20 adapted to detect the internal pressure of the working chamber 2. The pressure sensor 20 is adapted to detect the internal pressure of the working chamber 2 by measuring the internal pressure of an extending pipe 21 extending outward from the working chamber 2 so as to communicatively connect to the working chamber 2, and the pressure sensor 20 is electrically connected to the control part 10.

The extending pipe 21 is provided with decomposition means 22 on the upstream side of the pressure sensor 20. The decomposition means 22 is one having an ozone gas decomposition catalyst, and adapted to decompose the ozone gas to thereby prevent the ozone gas from flowing out to the downstream side of the decomposition means 22. Also, the extending pipe 21 is provided with an on-off valve 23 including a solenoid valve on the downstream side of the pressure sensor 20, and the on-off valve 23 is electrically connected to the control part 10.

Further, outside the cabinet main body 1, an ozone monitoring sensor 25 is provided, and the ozone monitoring sensor 25 is electrically connected to the control part 10.

Still further, outside the cabinet main body 1, an external exhaust fan P3 is provided, and the external exhaust fan P3 is electrically connected to the control part 10. The control part 10 is adapted to, when the ozone monitoring sensor 25 detects ozone gas outside the cabinet main body 1, open the exhaust valve B2 and drive the external exhaust fan P3 to exhaust the ozone gas to the outside of a building.

Also, the cabinet main body 1 is provided with airtightly closing means 30 that allows the shutter 4 to airtightly open/close the opening part 3.

That is, as illustrated in Fig. 2A to Fig. 2C, among the four circumferential parts of the opening part 3 of a rectangular shape, side edge parts along the left and right sides and a lower edge part along the lower side are provided with cross-sectionally U-shaped shutter rails 31, whereas as illustrated in FIG. 2C, an upper edge part along the upper side is provided with a horizontal rail 34 that is parallel separated from the shutter 4, and the horizontal rail 34 is provided with a pressing mechanism 35. The pressing mechanism 35 includes: a cross-sectionally L-shaped frame 35a attached on the lower surface of the horizontal rail 34; a shaft part 35b screwed into a screw hole provided in the frame 35a; and a touch part 35c provided at a fore end part of the shaft part 35b, and the touch part 35c is adapted to touch or separate from the shutter 4 in such a way that an operator rotates the shaft part 35b around the shaft center.

In addition, the left and right side parts and lower side part of the rectangular-shaped shutter 4 are inserted into the cross-sectionally U-shaped shutter rails 31, and the upper side part of the shutter 4 is inserted on an inner side than the touch part 35c. As a result, the shutter 4 is vertically slidable along the shutter rails 31 positioned at the left and right side edge parts, and movable in the thickness direction of the shutter 4 by the touch part 35c with the lower end part of the shutter 4 as a fulcrum.

In addition, in a state where the lower side part of the shutter 4 is inserted into the shutter rail 31 positioned at the lower edge part, the opening part 3 is adapted to be closed by the shutter 4, whereas the opening part 3 is adapted to open when the shutter 4 slides upward by a predetermined distance.

Also, between the right and left opposite shutter rails 31 and 31, between the shutter rail 31 on the lower side and the touch part 35c on the upper side, and on the inner side than the shutter 4, rectangular frame-shaped holding frames 32 formed in a cross-sectionally U-shape are provided with opening parts of the holding frames 32 facing to the shutter 4 side. In the holding frames 32, ring-shaped inflatable seals 33 that extend in the circumferential directions of the holding frames 32 are inserted.

The inflatable seals 33 are ones that are inflated by putting low-pressure air into tubular rubber seals, and in an inflated state, as illustrated in Fig. 2B and Fig. 2C, the opening part 3 is adapted to be airtightly closed by the shutter 4 in such a way that the inflatable seals 33 closely contact with the outer circumferential part of the back surface of the shutter 4 and the inflation of the inflatable seals 33 brings the outer circumferential part of the front surface of the shutter 4 into close pressure contact with the outer parts of the cross-sectionally U-shaped shutter rails 31 and with the touch part 35c of the pressing mechanism 35.

Also, in the case where the inflatable seals 33 are inflated, by rotating the shaft part 35b of the pressing mechanism 35, the touch part 35c presses the shutter 4 to, on the basis of the principle of leverage, allow the shutter 4 to move toward the upper side holding frame 32 with the lower end part of the shutter 4 as a fulcrum, and the back surface of the shutter 4 closely contact with the inflatable seals 33.

As described, by inflating the inflatable seals 33 to keep the shutter 4 so as to make the opening part 3 of the working chamber 2 unopenable, the shutter 4 can be prevented from being unexpectedly opened.

Meanwhile, when the inflatable seals 33 are deflated by removing air from the inflatable seals 33, as illustrated in Fig. 2A, the inflatable seals 33 are separated from the outer circumferential part of the back surface of the shutter 4 to release the pressure contact state of the shutter 4 with the shutter rails as well, and thereby the shutter 4 is made slidable upward.

Next, the actions of the safety cabinet (hereinafter abbreviated to BSC) having such a configuration will be described.

### (1) BSC operation flow

Fig. 3 is a flowchart for explaining a BSC operation flow.

First, in Step S1, when decontamination/BSC is in a stop state, i.e., when the ozone generator 13 and the air supply and circulating fan P1 are in a stop state, in Step S2, a shutter opening level is set to a setting value. That is, by raising the shutter 4, the shutter 4 is opened so as to make the distance (the shutter height) between the lower edge of the shutter 4 and the lower edge of the opening part 3 equal to a predetermined distance (a setting distance).

Note that when the shutter opening level exceeds the setting value, the air supply and circulating fan P1 is not started.

Then, in Step S3, an operation switch is pressed. As a result, in Step S4, the exhaust valve B2 opens.

Subsequently, in Step S5, the control part 10 determines whether the exhaust valve B2 opens, and when the exhaust valve B2 does not open, after confirming details, the flow returns to Step S4.

Meanwhile, when the exhaust valve B2 opens, in Step S6, the control part 10 starts the air supply and circulating fan P1 and the external exhaust fan P3. The start of the external exhaust fan P3 allows ambient air drawn into the safety cabinet to be exhausted to the outside of the building.

After that, in Step S7, the control part 10 determines whether the air supply and circulating fan P1 and the external exhaust fan P3 are driven, and when the air supply and circulating fan P1 and the external exhaust fan P3 are not driven, after confirming details, the flow returns to Step S4.

Meanwhile, when the air supply and circulating fan P1 and the external exhaust fan P3 are driven, in Step S8, the air supply and circulating fan P1 is continued to be driven for approximately one minute to perform cleanup operation for an approximately one minute.

Subsequently, in Step S9, a state where work in the working chamber 2 is startable is obtained, and therefore after that, the work in the working chamber 2 is performed.

Such work in the working chamber 2 is performed in a circulating manner.

### (2) BSC stop flow

FIG. 4 is a flowchart for explaining a BSC stop flow.

First, in Step S1, when the BSC is in an operation state, in Step S2, a fan switch is held down for approximately three seconds.

As a result, in Step S3, the air supply and circulating fan P1 is continued to be driven to perform the cleanup operation for approximately one minute after the end of the work.

Then, in Step S4, the control part 10 stops the air supply and circulating fan P1, and after that, in Step S5, the shutter 4 is closed, thus finally obtaining a BSC stop state in Step S6.

### (3) BSC decontamination operation flow

FIG. 5 is a flowchart for explaining a BSC decontamination operation flow.

First, in Step S1, when the decontamination/BSC is in the stop state, i.e., when the ozone generator 13, the air supply and circulating fan P1, and the external exhaust fan P3 are in the stop state, in Step S2, it is determined whether the shutter 4 closes. When the shutter 4 opens, in Step S3, the shutter 4 is closed, and the flow returns to Step S2.

In Step S2, when the shutter 4 closes (is in a fully closed state), in Step S4, a decontamination switch is pressed.

When pressing the decontamination switch, the control part 10 closes the exhaust valve B2.

Also, by introducing air into the inflatable seals 33 to pressurize the inflatable seals 33, the opening part 3 is airtightly closed by the shutter 4. In Step S5, a pressurized state of the inflatable seals 33 is checked because the pressurization of the inflatable seals 33 should be done at 60 to 70 kPa as a target, and when the pressurization is insufficient, after an immediate stop and cause investigation, the flow returns to Step S4, where the decontamination switch is pressed again.

In Step S5, when the pressurization of the inflatable seals 33 is sufficient, since the exhaust valve B2 closes, in Step S6, the airtightness level of the working chamber 2 is measured with the compressor of the oxygen generator 13a started and the air supply and circulating fan P1 stopped. That is, the on-off valve 23 is closed and then the airtightness level of the working chamber 2 is measured by the pressure sensor 20. In this case, the working chamber 2 is pressurized by the compressor to 300 to 500 Pa and then kept.

Then, in Step S7, it is checked whether the airtightness level of the working chamber 2 can be kept at 90 % or more of the predetermined pressure for 1 to 30 minutes, and when the airtightness level fails, after an immediate stop and cause investigation, the flow returns to Step S4, where the decontamination switch is pressed again.

Meanwhile, in Step S7, when the airtightness level passes of the working chamber 2 passes, in Step S8, after opening the supply valve 13d, the ozone generator 13 is started to introduce (supply) ozone gas into the working chamber 2, and also the humidifier 17 is started. This causes the concentration of ozone in the working chamber 2 to increases and also humidity to increase.

Subsequently, in Step S9, the concentration of ozone in the working chamber 2 is measured by the concentration sensor 15. When the concentration of ozone does not reach the lowest setting value such as 200 ppm, after an immediate stop and cause investigation, the flow returns to Step S4, where the decontamination switch is pressed again.

Meanwhile, in Step S9, it is confirmed that the concentration of ozone has the lowest setting value such as 200 ppm, and then, in Step S10, the humidity of the working chamber 2 is measured by the thermo-hygro sensor 16. When the humidity of the working chamber 2 does not reach 80 %, after an immediate stop and cause investigation, the flow returns to Step S4, where the decontamination switch is pressed again.

Also, in Step S10, it is confirmed that the humidity of the working chamber 2 is 80 % or more, and then the accumulative calculation of the below-described decontamination CT value is started. Then, in Step S11, it is determined whether the ozone generator 13 is normally driven, and when the ozone generator 13 is not normal, after an immediate stop and cause investigation, the flow returns to Step S4, where the decontamination switch is pressed again.

Meanwhile, in Step S11, when the ozone generator 13 is normally driven, in Step S12, it is constantly determined whether the ozone gas leaks out.

In this case, the ozone monitoring sensor 25 monitors the concentration of ozone outside the BSC, and when the concentration of ozone exceeds a predetermined value, the ozone generator 13 is immediately stops, the external exhaust fan P3 is started, and the exhaust valve B2 is opened to exhaust the ozone gas. Then, after cause investigation, the flow returns to Step S4, where the decontamination switch is pressed again.

Meanwhile, in Step S12, when it is determined that the concentration of ozone is equal to or more than the predetermined value, and the ozone gas does not leak out, in Step S13, the working chamber 2 of the BSC is decontaminated as "during normal decontamination".

### (4) Automatic BSC decontamination stop flow

Fig. 6 is a flowchart for explaining an automatic BSC decontamination stop flow.

When after decontaminating the working chamber 2 of the BSC as "during normal decontamination" as described above, the decontamination of the working chamber 2 ends, the decontamination of the BSC is automatically stopped in the following manner.

That is, on the basis of the ozone concentration of the ozone gas detected by the concentration sensor 15 and the CT value, the control part 10 performs control so as to automatically stop the ozone generator 13.

Here, the CT value will be described.

The CT value refers to the product of the ozone concentration (ppm) in the working chamber 2 and a decontamination time (minute), and is typically used as a target for the decontamination action of ozone.

Also, a target CT value is preliminarily set for each medicine or each germ as a "set CT value", and the set CT value is compared with the product of actually measured concentration and an elapsed time in an actual decontamination process and used to determine the end of the decontamination process. The set CT value is determined depending on the ozone resistance level of a medicine, a germ, or the like as a decontamination processing target, and in addition, when using a gaseous decontamination agent other than ozone gas, a corresponding set CT value is used.

As illustrated in Fig. 6, in Step S1, during the normal decontamination, the control part 10 samples an ozone concentration output from the concentration sensor 15, and after that, the control part 10 integrates a CT value from the sampled ozone concentration to determine whether the integrated CT value reaches the set CT value in Step S2.

Here, the integration of a CT value performed by the control part 10 will be described.

Simultaneously with starting the ozone generator 13, the control part 10 resets the value of an internal timer. Also, the control part 10 resets a CT value (Sct) for which a storage area is allocated in an unillustrated storage part (Sct = 0).

Subsequently, the control part 10 resets the value Ts of a sampling timer for managing an ozone concentration sampling interval (Ts = 0).

After that, every time a time Te (minute) preliminarily set as a sampling interval passes (to ≥ Te), the control part 10 samples ozone concentration, and adds the product of an actual sampling interval Ts (minute) and the sampled ozone concentration Co (ppm) to the CT value Sct.

The sampling interval Te is set to, for example, 0.5 to 5 seconds, but not limited to this.

Every time the product of the sampling interval Ts and the sampled ozone concentration is added to the CT value Sct, the control part 10 compare an updated CT value Sct and the set CT value Ect. As a result of the comparison, when the CT value Sct is equal to or less than the set CT value Ect, the value Ts of the sampling timer is reset and the ozone concentration sampling and the like are repeated.

Then, in Step S2, as a result of the comparison, when the CT value Sct is equal to or more than the set CT value Ect, for example, when the CT value Sct reaches, for example, 15000 that is the set CT value Ect, in Step S3, the control part 10 stops the ozone generator 13.

After that, in Step S4, the working chamber 2 is decomposed by a circulating air current using the air supply and circulating fan P1. Alternatively, only the compressor of the oxygen generator 13a is operated to decompose the ozone gas by the decomposition means 22, and by opening the on-off valve 23 to continuously discharge the decomposed gas, the ozone gas concentration is reduced.

Then, in Step S5, the ozone concentration in the working chamber 2 is measured by the concentration sensor 15, and for example, when the ozone concentration is 1 ppm or more, the flow returns to Step S4 with the shutter 4 closed.

Meanwhile, when the ozone concentration falls below 1 ppm, after manually opening the shutter 4, in Step S6, the air supply and circulating fan P1 and the external exhaust fan P3 are driven, and the exhaust valve B2 is opened to take the outside air into the working chamber 2, and in Step S7, a few minutes later, all are stopped.

### (5) Forcible BSC decontamination stop flow

Fig. 7 is a flowchart for explaining a forcible BSC decontamination stop flow.

As described above, in Step S1, when while the working chamber 2 of the BSC is being decontaminated as "during normal decontamination", the decontamination is required to be forcibly stopped, in Step S2, the decontamination switch is held down for, for example, approximately three seconds.

As a result, in Step S3, the control part 10 stops the ozone generator 13.

After that, in Step S4, the working chamber 2 is decomposed by a circulating air current using the air supply and circulating fan P1. Alternatively, only the compressor of the oxygen generator 13a is operated to decompose the ozone gas by the decomposition means 22, and by opening the on-off valve 23 to continuously discharge the decomposed gas, the ozone gas concentration is reduced.

Then, in Step S5, the ozone concentration in the working chamber 2 is measured by the concentration sensor 15, and for example, when the ozone concentration is 1 ppm or more, the flow returns to Step S4 with the shutter 4 closed.

Meanwhile, when the ozone concentration falls below 1 ppm, after manually opening the shutter 4, in Step S6, the air supply and circulating fan P1 and the external exhaust fan P3 are driven, and the exhaust valve B2 is opened to take the outside air into the working chamber 2, and in Step S7, a few minutes later, all are stopped.

As described above, according to the present embodiment, when decontaminating the working chamber 2 with ozone gas, the control part 10 closes the exhaust valve B2 and the airtightly closing means 30 makes the shutter 4 airtightly close the opening part 3, and then when the control part 10 controls the ozone generator 13 to introduce ozone gas into the working chamber 2 as well as driving the air supply and circulating fan P1, since the exhaust valve B2 is closed and also the opening part 3 is airtightly closed, the ozone gas introduced into the working chamber 2 circulates in the working chamber 2 without leaking out of the working chamber 2.

Accordingly, during the decontamination with the ozone gas, the ozone gas can be prevented from leaking out of the working chamber 2, and the ozone gas can be circulated in the working chamber 2 to decontaminate the working chamber 2 with the ozone gas.

Also, since the concentration sensor 15 adapted to detect the concentration of ozone in the working chamber 2 is provided connected to the control part 10, and on the basis of the ozone concentration detected by the concentration sensor 15, the control part 10 controls the ozone generator 13, the working chamber 2 can be decontaminated with a predetermined concentration of the ozone gas.

Further, since the control part 10 controls the ozone generator 13 on the basis of a CT value that is the product of the ozone concentration detected by the concentration sensor 15 and a decontamination time, when the CT value is equal to or more than a set CT, the control part 10 can stop the ozone generator 13. Accordingly, the working chamber 2 can be decontaminated with the ozone gas in an appropriate time.

Also, since the airtightly closing means 30 has the inflatable seals 33, by inflating the inflatable seals 33, the opening part 3 communicatively connecting to the working chamber 2 can be airtightly closed by the shutter 4, whereas by deflating the inflatable seals 33, the shutter 4 can be easily raised from the opening part 3 to open the opening part 3.

Further, since by inflating the inflatable seals 33, the inflatable seals 33 closely contact with the shutter 4, and also the outer circumferential part of the front surface of the shutter 4 is brought into airtight pressure contact with the shutter rails 31, the opening part 3 can be surely airtightly closed.

Also, since the pressure sensor 20 adapted to detect the internal pressure of the working chamber 2 is provided connected to the control part 10, before decontaminating the working chamber 2 with the ozone gas, the control part 10 closes the exhaust valve B2 as well as driving the compressor of the oxygen generator 13a to thereby raise the internal pressure of the working chamber 2, and the raised internal pressure is detected by the pressure sensor 20.

In addition, when the pressure sensor 20 detects that the internal pressure of the working chamber 2 is kept at a predetermined internal pressure for a predetermined time, the control part 10 can start the ozone generator 13 to introduce ozone gas into the working chamber 2. Accordingly, the initial leakage of ozone gas can be prevented.

Also, since the shutter 4 allowing opening/closing of the opening part 3 of the working chamber 2 can be kept unopenable by inflating the inflatable seals 33, when the concentration of the ozone gas in the working chamber 2 is equal to or more than a predetermined value, it is impossible for an operator to carelessly open the shutter 4, thus being superior in safety.

Note that the present embodiment has been described by taking as an example the case of using ozone gas as the gaseous decontamination agent; however, without limitation to ozone gas, the present invention may use another gaseous decontamination agent having a decontamination effect, such as a hydrogen peroxide gas or a chlorine dioxide gas.

Also, in the present embodiment, the ozone generator 13 is provided in the cabinet main body 1; however, the ozone generator 13 may be provided separately from the cabinet main body 1 and adapted to be detachably connectable to the cabinet main body 1. Reference Signs List

1 Cabinet main body
2 Working chamber
3 Opening part
4 Shutter (opening/closing member)
6 Exhaust path
10 Control part
13 Ozone generator (decontamination agent introduction means)
15 Concentration sensor
20 Pressure sensor
30 Airtightly closing means
B2 Exhaust valve
P1 Air supply and circulating fan
P3 External exhaust fan

## Claims

1. A safety cabinet comprising: a cabinet main body that has a working chamber inside; an opening/closing member that is provided at a front of the cabinet main body and allows opening/closing of an opening part communicatively connecting to the working chamber; and an exhaust path through which gas is exhausted from the working chamber, wherein the gas in the working chamber is made internally circulatable, the safety cabinet comprising:
an exhaust valve that is provided in the exhaust path;
an air supply and circulating fan that is provided in the working chamber;
decontamination agent introduction means that introduces a gaseous decontamination agent into the working chamber;
a control part that controls the exhaust valve, the air supply and circulating fan, and the decontamination agent introduction means; and
airtightly closing means that allows the opening/closing member to airtightly close the opening part.

2. The safety cabinet according to claim 1, wherein a concentration sensor adapted to detect concentration of the gaseous decontamination agent in the working chamber is provided connected to the control part, and on a basis of the concentration of the gaseous decontamination agent, the concentration being detected by the concentration sensor, the control part controls the decontamination agent introduction means.

3. The safety cabinet according to claim 2, wherein the control part controls the decontamination agent introduction means on a basis of a CT value that is a product of the concentration of the gaseous decontamination agent, the concentration being detected by the concentration sensor, and a decontamination time.

4. The safety cabinet according to any one of claims 1 to 3, wherein
the airtightly closing means has an inflatable seal.

5. The safety cabinet according to any one of claims 1 to 4, wherein:
the decontamination agent introduction means is configured to be able to introduce air into the working chamber; and
a pressure sensor adapted to detect internal pressure of the working chamber is provided connected to the control part.

6. A method for decontaminating the safety cabinet according to any one of claims 1 to 5, the method
using the airtightly closing means to airtightly seal the opening part of the working chamber of the safety cabinet by the opening/closing member as well as closing the exhaust valve, and after an airtightness level of the working chamber has become a predetermined value or more, introducing the gaseous decontamination agent into the working chamber by the decontamination agent introduction means; and
in a state where the concentration of the gaseous decontamination agent rises to a predetermined value, decontaminating the working chamber with the gaseous decontamination agent.

7. The method for decontaminating the safety cabinet, according to claim 6, the method
when the CT value of the gaseous decontamination agent in the working chamber reaches a predetermined value, stopping the introduction of the gaseous decontamination agent.

8. The method for decontaminating the safety cabinet, according to claim 6 or 7, the method
when the concentration of the gaseous decontamination agent in the working chamber is equal to or more than the predetermined value, inflating the inflatable seal to thereby keep unopenable the opening/closing member allowing opening/closing of the opening part communicatively connecting to the working chamber.
